(19) Europäisches Patentamt
European Patent Office
Office européen des brevets

(11) Numéro de publication: **0 345 116 B1**

# (12) FASCICULE DE BREVET EUROPEEN

(45) Date de publication de fascicule du brevet: **06.10.93**

(51) Int. Cl.[5]: **C09K 3/14**, A61K 7/16, C09C 1/30

(21) Numéro de dépôt: **89401398.6**

(22) Date de dépôt: **23.05.89**

## (54) Silice pour compositions dentifrices compatible notamment avec le zinc.

(30) Priorité: **01.06.88 FR 8807279**

(43) Date de publication de la demande:
**06.12.89 Bulletin 89/49**

(45) Mention de la délivrance du brevet:
**06.10.93 Bulletin 93/40**

(84) Etats contractants désignés:
**AT BE CH DE ES FR GB GR IT LI LU NL SE**

(56) Documents cités:
**EP-A- 0 046 057**
**DE-A- 2 544 218**
**FR-A- 1 327 033**
**US-A- 4 144 321**

**CHEMICAL ABSTRACTS, vol. 108, no. 22, 30 mai 1988, page 88, résumé no. 188517z, Columbus, Ohio, US; & SU-A-1 366 520**

(73) Titulaire: **RHONE-POULENC CHIMIE**
**25, quai Paul Doumer**
**F-92408 Courbevoie Cédex(FR)**

(72) Inventeur: **Persello, Jacques**
**14 Chemin de Calisse**
**310 - La Boisse 01120 - Montluel(FR)**

(74) Mandataire: **Dutruc-Rosset, Marie-Claude et al**
**RHONE-POULENC CHIMIE**
**Service Brevets Chimie**
**25, Ouai Paul Doumer**
**F-92408 Courbevoie Cédex (FR)**

EP 0 345 116 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne une silice utilisable notamment dans les compositions dentifrices, son procédé de préparation ainsi que des compositions dentifrices comprenant cette silice.

On sait que la silice est couramment utilisée dans la préparation de compositions dentifrices. Elle peut y jouer d'ailleurs plusieurs rôles.

Elle agit tout d'abord comme agent abrasif en aidant par son action mécanique à l'élimination de la plaque dentaire.

Elle peut aussi jouer le rôle d'agent épaississant pour conférer des propriétés rhéologiques déterminées au dentifrice ainsi que d'agent optique pour lui donner la coloration souhaitée.

Par ailleurs, on sait que les dentifrices contiennent des agents divers notamment pour la prévention des caries, pour diminuer la formation de la plaque dentaire ou le dépôt de tartre sur les dents. Parmi ces agents on peut citer en particulier le zinc. D'autres éléments sont aussi utilisés tels les fluorures phosphates, les pyrophosphates, les polyphosphates, les polyphosphonates, les guanidines, notamment les bis-biguanides dont l'un des éléments le plus employé est la chlorhexidine. Les formulations dentifrices peuvent aussi comporter des aromes, des parfums, etc...

La présence de ces agents dans le dentifrice pose le problème de leur compatibilité avec la silice. En effet à cause notamment de ses capacités absorbantes, celle-ci peut avoir tendance à réagir avec ces agents de telle sorte qu'ils ne soient plus disponibles pour exercer les effets thérapeutiques décrits plus haut.

L'objet de l'invention est donc de trouver des silices compatibles avec les agents mentionnés ci-dessus notamment avec le zinc et donc parfaitement utilisables dans la formulation de dentifrices.

Un autre objet de l'invention est un procédé permettant la préparation de tels silices compatibles.

Or, la Demanderesse s'est aperçu que les propriétés de compatibilité de la silice dépendaient de la chimie de surface de celle-ci. Plus particulièrement, la présence de zinc sur la surface de la silice, présence obtenue par un traitement spécifique de cette silice, rend cette dernière compatible avec cet élément.

De ce fait, la silice de l'invention est caractérisée en ce qu'elle comporte du zinc à sa surface et lié chimiquement à cette surface.

L'invention concerne aussi le procédé de préparation d'une telle silice compatible avec le zinc. Ce procédé est caractérisé en ce qu'on fait subir à la silice, soit préalablement préparée, soit lors d'une étape de sa préparation un traitement avec un zincate et, conjointement ou postérieurement à ce premier traitement, un traitement acide.

Enfin, l'invention est aussi relative aux compositions dentifrices à base d'une silice du type décrit ci-dessus ou du type préparé par le procédé qui vient d'être mentionné.

Les silices de l'invention présentent une compatibilité avec le zinc particulièrement élevée, en particulier d'au moins 50 %.

D'autres caractéristiques et avantages de l'invention seront mieux compris à la lecture de la description et des exemples non limitatifs qui vont suivre.

Comme cela a été vu plus haut, la caractéristiques essentielle de la silice de l'invention est sa chimie de surface. Cette silice comporte du zinc en surface et ce zinc est lié chimiquement à cette surface.

L'existence de cette liaison chimique peut être appréhendé par le comportement de la silice lorsqu'elle est mise dans une suspension aqueuse, dans l'eau par exemple, à pH 7. Dans ce cas, on n'observe pas de désorption du zinc ou qu'une désorption négligeable due à des traces de zinc non lié chimiquement, ces traces pouvant résulter d'une préparation à une pureté moins poussée de la silice.

Sans vouloir être lié ou limité par une théorie particulière, on peut estimer que cette liaison est problablement du type Si-O-Zn et plus précisément, qu'on doit avoir à la surface de la silice des groupements du type

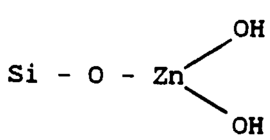

ou Si - O - Zn - OH

Enfin, il y a lieu de noter que c'est le zinc en tant que tel qui participe à la liaison Si-O-Zn, ceci contrairement à certaines silices de l'art antérieur dont la surface peut comporter du zinc, sous la forme

sulfate de zinc par exemple, non chimiquement lié ou adsorbé de façon réversible.

Comme on l'a vu plus haut la silice de l'invention présente une compatibilité avec le zinc d'au moins 50 %. Il est à noter que selon des modes de réalisation préférés de l'invention cette compatibilité peut aller jusqu'à au moins 80 % et même au moins 95 %. Cette compatibilité est mesurée par un test qui sera décrit plus loin.

La teneur en zinc de la silice peut varier en fonction de la surface spécifique de la silice et aussi de son application. Généralement, on préfère limiter cette quantité à 10 % exprimée en poids, de Zn par rapport à $SiO_2$. Cette limite peut être ramenée à 5% de Zn. Selon certains modes de réalisation, cette quantité est d'au plus 1 % et plus particulièrement d'au plus 0,5 %.

Une caractéristique nouvelle et particulièrement intéressante de la silice de l'invention réside dans le fait que, contrairement à l'art antérieur, cette silice peut présenter en même temps une compatibilité avec le zinc nettement augmentée tout en ayant une teneur en cet élément réduit. Ainsi la compatibilité de 50 % évoquée plus haut peut être obtenue avec une silice en teneur en zinc d'au plus 5%.

Outre les caractéristiques de chimie de surface qui viennent d'être décrites ci-dessous et qui conditionnent les compatibilités, les silices de l'invention présentent aussi des caractéristiques physiques qui les rendent parfaitement adaptées à leur application en dentifrice. Ces caractéristiques de type structurel vont être décrites ci-dessous.

Généralement la surface BET des silices de l'invention est comprise entre 40 et 600 $m^2/g$. Leur surface CTAB varie habituellement entre 40 et 400 $m^2/g$.

La surface BET est déterminée selon la méthode de BRUNAUER-EMMET-TELLER décrite dans the Journal of the American Chemical Society vol. 60, page 309, February 1938 et selon la norme NF T45 007-(5.11).

La surface CTAB est la surface externe déterminée selon la norme NF T45 007 (5.12).

Les silices de l'invention peuvent bien entendu correspondre aux trois types habituellement distingués dans le domaine du dentifrice.

Ainsi, les silices de l'invention peuvent être du type abrasif. Elles présentent alors une surface BET comprise entre 40 et 300 m2/g. Dans ce cas, la surface CTAB est comprise entre 40 et 100 m2/g.

Les silices de l'invention peuvent aussi être du type épaississant. Elles ont alors une surface BET comprise entre 120 et 450 m2/g plus particulièrement 120 et 300 m2/g. Elles pouront présenter alors une surface CTAB entre 120 et 400 m2/g.

Enfin, selon un troisième type, les silices de l'invention peuvent être bifonctionnelles. Elles possèdent ici une surface BET comprise entre 80 et 200 m2/g. La surface CTAB est alors comprise entre 80 et 200 m2/g.

Les silices de l'invention peuvent aussi présenter une prise d'huile comprise entre 80 et 500 cm3/100 g déterminée selon la norme NFT 30-022 (mars 1953) en mettant en oeuvre le phtalate de dibutyle.

Plus précisément, cette prise d'huile sera comprise entre 100 et 140 $cm^3/100$ g pour les silices abrasives, 200 et 400 pour les silices épaississantes et 100 et 300 pour les bifonctionnelles.

Par ailleurs, toujours en vue de l'application en dentifrice, les silices ont préférentiellement une taille de particules comprise entre 1 et 10 $\mu$m.

Le pH des silices mesuré selon la norme NFT 45-007 est généralement compris entre 6 et 10.

La densité apparente variera généralement entre 0,01 et 0,6.

Enfin, les silices de l'invention présentent un indice de réfraction compris entre 1,440 et 1,465.

Selon un mode de réalisation particulier de l'invention, les silices sont des silices de précipitation.

Le procédé de préparation des silices de l'invention va maintenant être décrit.

Ce procédé consiste à faire subir à la silice deux traitements : un premier par un zincate, un second qui est un traitement acide. Ces deux traitements peuvent se faire l'un après l'autre ou conjointement.

On entend par zincate les composés contenant les anions $ZnO_2^{2-}$, $HZnO_2^{-}$, $Zn_2O_4^{4-}$, $ZnO_4^{6-}$.

On utilise plus particulièrement les zincates alcalins et notamment le zincate de sodium.

Ces zincates peuvent être préparés par réaction de solutions alcalines avec le zinc ou un oxyde de zinc.

D'une manière générale, le traitement zincate se fait par mise en contact de la silice ou d'une suspension de silice avec un zincate, habituellement sous forme d'une solution.

Le traitement acide, d'une manière générale, se fait par passage d'une solution acide sur la silice ou addition de cette solution à une suspension contenant la silice.

Dans ce dernier cas, on ajoute une quantité de solution acide telle que le pH de la suspension après le traitement, c'est-à-dire après l'ajout de la solution, ait une valeur qui ne soit pas inférieure à 7.

Cette solution acide peut être par exemple une solution d'un acide inorganique tel que l'acide nitrique, l'acide sulfurique, l'acide chlorhydrique, l'acide carbonique.

Toutefois, cette solution acide peut aussi être une solution d'un acide organique. Il faut cependant veiller à ce que cet acide ne soit pas susceptible de former des complexes avec le zinc.

Le procédé qui vient d'être décrit d'une manière générale va être étudié plus particulièrement ci-dessous.

Il y a tout d'abord lieu de noter que les traitements ci-dessus peuvent être effectués durant la préparation même de la silice et ceci à une étape quelconque de cette préparation ou bien sur une silice préalablement préparée.

La silice traitée de l'invention peut être préparée par tout moyen connu. Ce peut être une silice de précipitation obtenue par un procédé du type comprenant la réaction d'un silicate avec un agent acidifiant ce qui donne lieu à la formation d'une suspension ou d'un gel de silice.

Il est à noter que l'on peut utiliser tout mode opératoire connu pour arriver à cette suspension ou ce gel (Addition d'acide sur un pied de cuve de silicate, addition simultanée totale ou partielle d'acide et de silicate sur un pied de cuve d'eau ou de suspension de silicate, mûrissement etc...), le choix se faisant essentiellement en fonction des caractéristiques physiques de la silice que l'on désire obtenir.

On procède alors à la séparation de la silice du milieu réactionnel selon tout moyen connu, filtre sous vide ou filtre presse par exemple.

On recueille ainsi un gâteau de silice, qui est lavé si nécessaire.

Ce gâteau, ou s'il délité, la suspension de délitage, est séché par tout moyen connu notamment par atomisation. Le produit séché est broyé si nécessaire pour obtenir la granulométrie désirée.

Selon la caractéristique principale du procédé de l'invention, on traite la silice avec un zincate et ce traitement peut se faire à une étape quelconque du procédé décrit ci-dessus.

Le zincate peut être introduit dans la suspension avant la filtration ou sur le gel de silice. Toutefois, dans le cas de l'obtention d'une suspension, on préfère introduire le zincate après l'étape de séparation-filtration.

Dans ce cas, le zincate est introduit sur le gâteau issu de cette séparation ou, plus généralement, dans la suspension obtenue après délitage de ce gâteau.

Le traitement acide peut se faire par addition d'une solution acide à la suspension avant la filtration ou sur le gel simultanément ou postérieurement au traitement du zincate. Là aussi toutefois, on préfère le faire, après la séparation, sur le gâteau ou le gâteau délité, par passage de la solution acide sur le gâteau, ou addition de cette solution à la suspension de délitage. De même le traitement acide peut se faire conjoitement ou postérieurement au traitement zincate.

Il peut être aussi intéressant de prévoir un mûrissement de la silice une fois celle-ci traitée.

Ce mûrissement se fait généralement à une température comprise entre 60 et 100 ° C. Bien entendu, en fonction de l'étape où le traitement au zincate est conduit, ce mûrissement pourra se faire sur la suspension directement obtenue par la réaction de précipitation ou sur le gel, ou bien sur le gâteau de filtration délité si le traitement au zincate a été fait sur la silice issue de la filtration.

Dans le cas d'une sicile préalablement préparée et éventuellement d'une silice autre qu'une silice de précipitation, les traitements qui ont été décrits ci-dessus peuvent être utilisés sans changement dans leur principe.

Ces traitements seront mis en oeuvre généralement en remettant la silice, en suspension et en ajoutant à la suspension ainsi formée le zincate et la solution acide de la même manière que ce qui a été décrit précédemment.

Une fois la silice traitée, celle-ci peut être lavée par exemple à l'eau déionisée puis séchée.

Généralement, le lavage se fait sur le gâteau issu de la filtration des suspensions précitées puis ce gâteau est délité pour séchage ultérieur. Généralement, on ajuste le pH de la suspension, cet ajustement fixant le pH de la silice finie.

Les procédés de préparation qui viennent d'être décrits mettent en oeuvre un zincate qui a été préparé préalablement au traitement de la silice.

Toutefois, il est possible dans le cadre de la présente invention de préparer le zincate "in situ". Dans ce cas-là, au lieu de réaliser le traitement avec un zincate tel que mentionné ci-dessus, on fait agir sur la silice un mélange comprenant un oxyde de zinc, un silicate et une base qui peut être par exemple un hydroxyde alcalin, alcalino terreux ou d'ammonium.

On effectue ensuite un traitement acide du type de celui qui a été décrit plus haut.

Une autre variante du procédé concerne la préparation de silices compatibles avec les guanidines et, en outre, d'autres éléments tels que le fluor et les phosphates.

Cette variante consiste essentiellement à laver suffisamment la silice après traitement. Ce lavage se fait habituellement sur le gâteau issu de la filtration des suspensions précitées jusqu'à ce qu'on obtienne un filtrat présentant une conductivité d'au plus 2000 microsiemens.$cm^{-1}$ plus particulièrement d'au plus 1000

microsiemens.$cm^{-1}$.

L'invention concerne aussi des compositions dentifrice contenant les silices du type décrit ci-dessus ou obtenues par le procédé qui vient d'être étudié.

La quantité de silice selon l'invention utilisée dans les compositions dentifrices peut varier dans de larges limites, elle est habituellement comprise entre 5 et 35% en poids.

Les silices de l'invention s'appliquent particulièrement bien aux compositions dentifrices contenant au moins un élément choisi dans le groupe comprenant les fluorures, les phosphates, les polyphosphates, les pyrophosphates et les polyphosphonates, le zinc, les guanidines.

En ce qui concerne les composés fluorés, leur quantité correspond de préférence à une concentration en fluor dans la composition comprise entre 0,01 et 1 % en poids et plus particulièrement 0,1% à 0,5 %. Les composés fluorés sont en particulier les sels de l'acide monofluorophosphorique et particulièrement ceux de sodium, potassium, lithium, calcium, aluminium et ammonium, le mono et le difluorophosphate ainsi que des fluorures variés contenant le fluor sous forme d'ion lié particulièrement les fluorures alcalins comme ceux de sodium, lithium, potassium, le fluorure d'ammonium, le flurorue stanneux, le fluorure de manganèse, le fluorure de zirconium, le fluorure d'aluminium ainsi que des produits d'addition de ces fluorures entre eux ou avec d'autres fluorures, tels que les fluorures de potassium ou de sodium ou de manganèse.

D'autres fluorures sont également utilisables pour la présente invention comme, par exemple, le fluorure de zinc, le fluorure de germanium, le fluorure de palladium, le fluorure de titane, les fluozirconates alcalins par exemple de sodium ou de potassium, le fluozirconate stanneux, le fluoborate ou les fluosulfates de sodium, de potassium.

Les composés fluorés organiques peuvent également être utilisés, de préférence ceux connus comme les produits d'addition d'amines ou d'amino-acides à longue chaîne avec le fluorure d'hydrogène, le fluorure de cétylamine, le dihydrofluorure de bis-(hydroxyéthyl)aminopropyl N-hydroxyéthyl octadécylamine, le fluorure d'octadécylamine et le dihydrofluorure de N, N′, N′tri-(polyoxyéthylène) N-hexadécylpropylènodiamine.

En ce qui concerne le zinc, celui-ci est présent généralement sous forme d'un sel soluble notamment sous forme citrate ou sulfate.

Pour les éléments utilisables comme agents anti-plaques du type polyphosphates ou polyphosphonates, on peut mentionner ceux indiqués dans le brevet US 3.934.002.

Les compositions dentifrices peuvent contenir en outre un liant.

Les principaux liants utilisés sont notamment choisis parmi :

- les dérivés cellulosiques : méthylcellulose, hydroxyéthylcellulose, carboxyméthylcellulose sodique,
- les mucilages : carraghénates, alginates, agar-agar et géloses,
- les gommes : gommes arabique et adragante, gomme xanthane, gomme Karaya,
- les polymères carboxyvinyliques et acryliques,
- les résines de polyoxyéthylène.

Outre les silices de l'invention, les compositions dentifrices peuvent contenir aussi un ou plusieurs autres agents abrasifs polissants choisis notamment parmi :

- le carbonate de calcium,
- le carbonate de magnésium,
- les phosphates de calcium, di-et tricalciques,
- le métaphosphate de sodium insoluble,
- le pyrophosphate de calcium,
- l'oxyde de titane (agent de blanchiment),
- les silicates,
- les alumines et silico-aluminates,
- les oxydes de zinc et d'étain,
- le talc,
- le kaolin.

Les compositions dentifrices peuvent aussi comprendre des détergents, des humectants, des agents aromatisants, édulcorants, des colorants et des conservateurs.

Les principaux détergents utilisés sont notamment choisis parmi :

- le laurylsulfate de sodium,
- le lauryléthersulfate et le laurysulfoacétate de sodium,
- le dioctylsulfosuccinate de sodium,
- le laurylsarcosinate de sodium,
- le ricinoléate de sodium,

- les monoglycérides sulfatés.

Les principaux agents humectants utilisés sont notamment choisis parmi les polyalcools comme :

- le glycérol,
- le sorbitol, généralement en solution à 70% dans l'eau,
- le propylène glycol.

Les principaux agents aromatisants (parfum) sont notamment choisis parmi : les essences d'anis, de badiane, de menthe, de baie de genièvre, de cannelle, de girofle et de rose.

Les principaux agents édulcorants sont notamment choisis parmi les imides orthosulfobenzoïques et les cyclamates.

Les principaux colorants utilisés sont notamment choisis selon la couleur désirée parmi :

- coloration rouge et rose : amaranthe, azorubine, cachou, coccine nouvelle (PONCEAU 4R), cochenille, érythrosine,
- coloration verte : chlorophylle et chlorophylline,
- coloration jaune : jaune soleil (Orange S) et jaune de quinoléine.

Les principaux conservateurs les plus utilisés sont : les parahydroxybenzoates, le formol et les produits qui en dégagent, l'héxétidine, les ammoniums quaternaires, l'hexachlorophène, le bromophène et l'hexamédine.

Enfin, les compositions dentifrices contiennent des agents thérapeutiques dont les principaux sont notamment choisis parmi :

- les antiseptiques et les antibiotiques,
- les enzymes,
- les oligo-éléments et les composés fluorés qui ont été décrits ci-dessus.

Des exemples concrets mais non limitatifs vont maintenant être donnés. Auparavant les tests pour la mesure de la compatibilité de la silice avec différents éléments vont être décrits.

Mesure de la compatibilité avec les fluorures

4 g de silice sont dispersés dans 16 g de solution à 0,3 % de fluorure de sodium (NaF). La suspension est agitée pendant 24 heures à 37 °C. Après centrifugation de la suspension à 20000 t/mn pendant 30 mn, le surnageant est filtré sur filtre Millipore 0,2 µm. La solution ainsi obtenue, constitue la solution de l'essai.

Une solution de référence est constituée en utilisant le même protocole mais en absence de silice.

La compatibilité avec les fluorures est déterminée par le % de fluorure libre mesuré par électrode sélective à fluorure (Orion). Elle est déterminée par la relation ci-dessous.

$$\text{\% Compatibilité} = \frac{\text{Concentration en F de l'essai (ppm)}}{\text{Concentration en F de la référence (ppm)}} \times 100$$

Mesure de la compatibilité avec le zinc

4 g de silice sont dispersés dans 100 ml de solution à 0,06 % de Zn $SO_4$, $7H_2O$. On obtient une suspension dont le pH est stabilisé à 7 pendant 15 minutes par ajout de NaOH ou $H_2SO_4$. La suspension est agitée ensuite pendant 24 heures à 37 °C puis est centrifugée à 20000 t/mn pendant 30 mn.

Le surnageant filtré sur filtre Millipore 0,2 µm, constitue la solution de l'essai.

Une solution de référence est constituée en suivant le même protocole en absence de silice.

La concentration en zinc libre des deux solutions est déterminée par absorption atomique (214 nm).

La compatibilité est déterminée par la relation ci-dessous :

$$\text{\% Compatiblité} = \frac{\text{Concentration en Zn de l'essai (ppm)}}{\text{Concentration en Zn de la référence (ppm)}} \times 100$$

Mesure de la compatibilité avec les pyrophosphates de sodium et de potassium

4 g de silice sont dispersés dans 16 g de suspension à 1,5 % de pyrophosphate de sodium ou de potassium. La suspension est agitée pendant 24 heures à 37 °C puis est centrifugée à 20000 t/mn pendant 30 mn.

Le surnageant est filtré sur filtre Millipore 0,2 µm.0,2g de solution diluée dans 100 ml d'eau une fiole jaugée, constitue la solution de l'essai.

Une solution de référence est constituée en suivant le même protocole mais en absence de silice.

La concentration en ion pyrophosphate ($P_2O_7^{--}$) libre des deux solutions est déterminée par chromatographie ionique (système DIONEX 2000i) équipé d'un intégrateur.

La compatibilité est déterminée par le rapport des aires des pics obtenus sur les chromatogrammes et correspondant au temps de rétention du pyrophosphate, de l'essai et de la référence.

$$\%\ \text{Compatibilité} = 100 \times \frac{\text{aire du pic de l'essai}}{\text{aire du pic de la référence}}$$

Mesure de la compatibilité avec la chlorhexidine

4 g de silice sont dispersés dans 16 g d'une solution aqueuse de chlorhexidine de concentration 1% en digluconate de chlorhexidine.

La suspension est agitée pendant 24 heures à 37 °C.

La suspension est ensuite centrifugée à 2000 t/mn pendant 30 mn et le surnageant obtenu est filtré sur filtre Millipore 0,2 µm.

Par la suite, 0,5 ml de solution ainsi filtrée est prélevé et dilué dans 100 ml d'eau dans une fiole jaugée. Cette solution constitue la solution de l'essai.

Une solution de référence est constituée selon le même protocole mais en absence de silice. Une solution aqueuse à 1 % de digluconate de chlorhexidine est agitée pendant 24 heures à 37 °C, puis est centrifugée à 2000 t/mn et le surnageant est filtré sur filtre Millipore de 0,2 µm. 0,5 ml de solution ainsi obtenue est dilué dans 100 ml d'eau dans une fiole jaugée.

On mesure ensuite l'absorbance des deux solutions à 254 nm à l'aide d'un spectrophotomètre (Uvikon 810/820).

La quantité de chlorhexidine libre notée % Compatibilité, est déterminée par le rapport :

$$\%\ \text{Compatibilité} = \frac{\text{Absorbance de l'essai}}{\text{Absorbance de la référence}} \times 100$$

EXEMPLE 1

Cet exemple décrit la préparation d'un zincate de sodium.

Dans un réacteur en verre à double enveloppe, muni d'une ancre pour une agitation lente, d'un réfrigérant à boule et chauffé par un bain d'huile, on ajoute 462 g de NaOH à 250 ml d'eau en pied de cuve.

On dissout et on monte en température.

A 65 °C, on ajoute 262 g de ZnO. On continue la montée en température sous reflux.

A 160 °C, après 1/2 heure, la suspension s'éclaircit et la dissolution est complète.

Après 1/2 heure à reflux à 160 °C, on laisse la suspension se refroidir. A environ 100 °C, on ajoute 575 ml d'eau.

A 70 °C, la solution a relargué et on ne peut redissoudre le zincate après 1 heure à 160 °C.
On filtre la suspension à 80 °C environ.
Le zincate obtenu a les caractéristiques suivantes :

| | |
|---|---|
| - Extrait sec | 48 % |
| - [Zn] 12,9 % soit | [ZnO] 16,1 % |
| | [$Na_2O$] 31,9 % |
| - ZnO / $Na_2O$ = | 0,39 molaire |
| - d = 1,59 | |

EXEMPLE 2

Dans un réacteur équipé d'un système de régulation de température et de pH et d'un système d'agitation par turbine, on introduit 6 l d'eau déionisée.

Après la mise en marche de l'agitation (300 t/mn), le pied de cuve ainsi constitué est chauffé à 85 °C.

Lorsque la température est atteinte, on procède à l'addition simultanée de 8,5 l de silicate de sodium de concentration en silice de 120 g/l, de rapport SiO2/Na2O égal à 3,5 et de débit 0,34 l/mn et 13,5 l d'acide sulfurique de concentration 80 g/l. Le débit d'acide est ajusté de manière à maintenir le pH du milieu à une valeur constante de 8,0.

Après 40 mn d'addition, on arrête l'addition de silicate et on continue l'addition d'acide jusqu'à stabiliser le pH du mélange réactionnel à 4.

On réaliser par la suite, un murissement de 15 mn à ce pH et à 85 °C.

Le mélange est ensuite filtré et le gâteau humide est lavé à l'eau déionisée.

Sur le gâteau délité, on ajoute en 30 mn, une solution contenant 40 g de zincate préparé selon l'exemple 1 et 60 g d'eau. Le pH est descendu à 8,3 par ajout d'acide chlorhydrique à 20 % et on laisse ensuite mûrir le mélange réactionnel pendant 15 mn à 80 °C.

Le mélange est ensuite filtré et le gâteau humide est lavé à l'eau déionisée jusqu'à ce que la conductivité du filtrat soit inférieure à 500 microsiemens.

Le gâteau est ensuite délité, puis on ajuste le pH de ce dernier à 6 par ajout d'acide acétique. Un dernier lavage est réalisé avec de l'eau déionisée.

Le produit est ensuite séché par atomisation et broyé sur un broyeur de type forplex pour obtenir une granulométrie de 8 microns.

Les caractéristiques physico-chimiques de la silice du type abrasive ainsi obtenue sont les suivantes :

| | |
|---|---|
| - Surface BET | 100 m2/g |
| - Surface CTAB | 80 m2/g |
| - Prise DOP | 120 ml/100 g |
| - Volume spécifique | 1,5 cm3/g |
| - pH à 5% dans l'eau | 7,5 |
| - Taux de Zinc par rapport à la silice | 0,5 % poids |

Dans le tableau ci-dessous, sont regroupées les différentes compatibilités de cette silice avec les ingrédients d'une formulation dentifrice et mesurées à l'aide des différents tests décrits ci-dessus :

| ingrédients | Fluorure NaF | Pyrophosphate Na/K | Chlorhexidine digluconate | Zinc ZnSO4 |
|---|---|---|---|---|
| % Compatible | 95 | 92 | 75 | 93 |

EXEMPLE 3

Dans le même réacteur que dans l'exemple 2, on constitue de la même manière un pied de cuve de 6 l.

Lorsque la température de 85 °C est atteinte, on ajuste le pH du milieu réactionnel à 9 par ajout de silicate de sodium à 90 g/l. On procède par la suite, à l'addition simultanée de silicate de sodium de concentration en silice de 90 g/l, de rapport $SiO_2/Na_2O$ égal à 3,5 avec un débit 0,090 l/mn et d'acide sulfurique de concentration 90 g/l pendant 20 mn. Le débit d'acide est ajusté de manière à maintenir le pH du milieu à une valeur constante de 9,0.

On ajoute ensuite 2 l de solution aqueuse de sulfate de sodium à 64 g/l et on continue l'addition simultanée de silicate de sodium et d'acide sulfurique pendant 40 mn;

On arrête l'addition de silicate et on continue l'addition d'acide jusqu'à stabiliser le pH du mélange réactionnel à 8.

On réalise par la suite, un murissement de 15 mn à ce pH et à 85 °C.

Le pH est ensuite descendu à 4 par ajout d'acide sulfurique. Le mélange est ensuite filtré et le gâteau humide est lavé à l'eau déionisée.

Sur le gâteau délité, on ajoute en 30 mn, une solution contenant 20 g de zincate préparé selon l'exemple 1 et 60 g d'eau. Le pH est descendu à 8,3 par ajout d'acide sulfurique à 20 % et on laisse ensuite mûrir le mélange réactionnel pendant 30 mn à 80 °C.

Le mélange est ensuite filtré et le gâteau humide est lavé à l'eau déionisée jusqu'à ce que la conductivité du filtrat soit inférieure à 500 microsiemens.

Le gâteau est ensuite délité, puis on ajuste le pH de ce dernier à 7 par ajout d'acide acétique.

Un dernier lavage réalisé avec de l'eau déionisée.

Le produit est ensuite séché par atomisation et micronisé par un broyeur de type "Jet Pulverizer" pour obtenir une granulométrie de 1,2 microns.

Les caractéristiques physico-chimiques de la silice du type épaississante ainsi obtenue sont les suivantes :

| | |
|---|---|
| - Surface BET | 220 m2/g |
| - Surface CTAB | 200 m2/g |
| - Prise DOP | 380 ml/100 g |
| - Volume spécifique | 8,0 cm3/g |
| - pH à 5% dans l'eau | 7,5 |
| - Taux de zinc par rapport à la silice | 0,5 % en poids |

Les compatibilités sont les suivantes :

| ingrédients | Fluorure NaF | Pyrophosphate Na/K | Chlorhexidine digluconate | Zinc ZnSO4 |
|---|---|---|---|---|
| % Compatible | 95 | 90 | 85 | 98 |

EXEMPLE 4

Cet exemple concerne la formulation d'une pâte dentifrice avec la silice de l'exemple 2. Les proportions sont données en poids :

| | |
|---|---|
| - Glycérine | 22 % |
| - Carboxyméthylcellulose 7mFD | 1,0 % |
| - Silice | 31,5 % |
| - $TiO_2$ | 1,0 % |
| - $ZnSO_4$, 7 $H_2O$ | 0,48 % |
| - Benzoate de sodium | 0,1 % |
| - Saccharinate de sodium | 0,2 % |
| - Monofluorophosphate de sodium | 0,76 % |
| - Fluorure de sodium | 0,1 % |
| - Laurylsulfate de sodium sol. à 30 %/eau | 4,66 % |
| - Arôme | 0,9 % |
| - Eau | 37,3 % |

Le dentifrice présente des propriétés rhéologiques satisfaisantes et s'extrude convenablement initialement et après stockage (2 mois). Une activité anti-bactérienne est constatée même après 2 mois de stockage à 37 °C.

Bien entendu, l'invention n'est nullement limitée aux modes de réalisation décrits qui n'ont été donnés qu'à titre d'exemples. En particulier, elle comprend tous les moyens constituant des équivalents techniques des moyens décrits ainsi que leurs combinaisons si celles-ci sont mises en oeuvre dans le cadre de la protection comme revendiquée.

**Revendications**
**Revendications pour les Etats contractants suivants : AT, BE, CH, DE, FR, GB, GR, IT, LI, LU, NL, SE**

**1.** Silice, compatible avec le zinc caractérisée en ce qu'elle comporte du zinc à sa surface et lié chimiquement à cette surface.

**2.** Silice selon la revendication 1, caractérisée en ce que lorsqu'elle est mise dans une solution aqueuse à pH 7, elle ne donne lieu qu'à une désorption nulle ou négligeable de zinc.

**3.** Silice selon la revendication 1 ou 2, caractérisée en ce qu'elle comporte à sa surface des groupements Si-O-Zn.

**4.** Silice selon l'une des revendications précédentes, caractérisée en ce qu'elle présente une compatibilité avec le zinc d'au moins 50 %.

**5.** Silice selon la revendication 4, caractérisée en ce qu'elle présente une compatibilité avec le zinc d'au moins 80 %, plus particulièrement d'au moins 95 %.

**6.** Silice selon l'une des revendications précédentes, caractérisée en ce qu'elle présente une teneur en zinc d'au plus 10%, plus particulièrement d'au plus 5 %.

**7.** Silice selon la revendication 6, caractérisée en ce qu'elle présente une teneur en zinc d'au plus 1 %, plus particulièrement d'au plus 0,5 %.

**8.** Silice selon l'une des revendications précédentes, caractérisée en ce qu'elle présente une surface BET comprise entre 40 et 600 $m^2/g$.

**9.** Silice selon l'une des revendications précédentes, caractérisée en ce qu'elle présente une surface CTAB comprise entre 40 et 400 $m^2/g$.

**10.** Silice selon l'une des revendications précédentes, du type abrasif caractérisée en ce qu'elle présente une surface BET comprise entre 40 et 300 $m^2/g$.

**11.** Silice selon la revendication 10, caractérisée en ce qu'elle présente une surface CTAB comprise entre 40 et 100 $m^2/g$.

12. Silice selon l'une quelconque des revendications 1 à 9, du type épaississant, caractérisée en ce qu'elle présente une surface BET comprise entre 120 et 450 $m^2/g$, plus particulièrement 120 et 300 $m^2/g$.

13. Silice selon la revendication 12, caractérisée en ce qu'elle présente une surface CTAB comprise entre 120 et 400 $m^2/g$.

14. Silice selon l'une quelconque des revendications 1 à 9, du type bifonctionnelle, caractérisée en ce qu'elle présente une surface BET comprise entre 80 et 200 $m^2/g$.

15. Silice selon la revendication 14, caractérisée en ce qu'elle présente une surface CTAB comprise entre 80 et 200 $m^2/g$.

16. Silice selon l'une des revendications précédentes, caractérisée en ce qu'elle présente une prise d'huile comprise entre 80 et 500 $cm^3/g$.

17. Silice selon l'une des revendications précédentes, caractérisée en ce qu'il s'agit d'une silice de précipitation.

18. Procédé d'obtention d'une silice compatible avec le zinc notamment du type selon l'une des revendications précédentes caractérisé en ce qu'on fait subir à la silice, soit préalablement préparée, soit lors d'une étape de sa préparation, un traitement avec un zincate et, conjointement ou postérieurement à ce premier traitement, un traitement acide.

19. Procédé selon la revendication 18, caractérisé en ce qu'on réalise le traitement avec un zincate par mise en contact de la silice ou d'une suspension de silice avec un zincate.

20. Procédé selon l'une des revendications 18 ou 19, caractérisé en ce que le traitement acide se fait par passage d'une solution acide sur la silice ou addition de cette solution à une suspension contenant la silice.

21. Procédé selon la revendication 20 caractérisé en ce que le traitement acide se fait par addition d'une solution acide à une suspension contenant la silice de manière que le pH de la suspension présente une valeur qui ne soit pas inférieure à 7 à la fin du traitement.

22. Procédé selon l'une des revendications 18 à 21 du type comprenant une réaction d'un silicate avec un agent acidifiant, l'obtention d'une suspension ou d'un gel de silice, une séparation et un séchage de la silice, caractérisé en ce qu'on traite avec un zincate le gâteau issu de la séparation de la silice de la suspension.

23. Procédé selon la revendication 22 caractérisé en ce qu'on traite le gâteau précité délité.

24. Procédé selon l'une des revendications 18 à 23 caractérisé en ce qu'on utilise un zincate alcalin.

25. Procédé selon l'une des revendications 18 à 24, caractérisé en ce qu'après le traitement par le zincate et le traitement acide on fait subir un mûrissement à la silice.

26. Composition dentifrice caractérisée en ce qu'elle comporte une silice du type selon l'une des revendications 1 à 17 ou du type préparée par le procédé selon l'une des revendications 18 à 25.

27. Composition dentifrice selon la revendication 26, caractérisée en ce qu'elle comporte du zinc.

28. Composition dentifrice selon la revendication 26 ou 27, caractérisée en ce qu'elle comporte au moins un élément choisi dans le groupe des fluorures, des phosphates, des polyphosphates, des polyphosphonates, des pyrophosphates, des guanidines.

**Revendications pour l'Etat contractant suivant : ES**

1. Procédé d'obtention d'une silice compatible avec le zinc, comportant du zinc à sa surface et lié chimiquement à cette surface, caractérisé en ce qu'on fait subir à la silice, soit préalablement préparée, soit lors d'une étape de sa préparation, un traitement avec un zincate et, conjointement ou postérieurement à ce premier traitement, un traitement acide.

2. Procédé selon la revendication 1, caractérisé en ce que lorsque ladite silice est mise dans une solution aqueuse à pH 7, elle ne donne lieu qu'à une désorption nulle ou négligeable de zinc.

3. Procédé selon la revendication 1 ou 2, caractérisé en ce que ladite silice comporte à sa surface des groupements Si-O-Zn.

4. Procédé selon l'une des revendications précédentes, caractérisé en ce que ladite silice présente une compatibilité avec le zinc d'au moins 50 %.

5. Procédé selon la revendication 4, caractérisé en ce que ladite silice présente une compatibilité avec le zinc d'au moins 80 %, plus particulièrement d'au moins 95 %.

6. Procédé selon l'une des revendications précédentes, caractérisé en ce que ladite silice présente une teneur en zinc d'au plus 10 %, plus particulièrement d'au plus 5 %.

7. Procédé selon la revendication 6, caratérisé en ce que ladite silice présente une teneur en zinc d'au plus 1 %, plus particulièrement d'au plus 0,5 %.

8. Procédé selon l'une des revendications précédentes, caractérisé en ce que ladite silice présente une surface BET comprise entre 40 et 600 $m^2/g$.

9. Procédé selon l'une des revendications précédentes, caractérisé en ce que ladite silice présente une surface CTAB comprise entre 40 et 400 $m^2/g$.

10. Procédé selon l'une des revendications précédentes, caractérisé en ce que ladite silice est du type abrasif et présente une surface BET comprise entre 40 et 300 $m^2/g$.

11. Procédé selon la revendication 10, caractérisé en ce que ladite silice présente une surface CTAB comprise entre 40 et 100 $m^2/g$.

12. Procédé selon l'une quelconque des revendications 1 à 9, caractérisé en ce que ladite silice est du type épaississant et présente une surface BET comprise entre 120 et 450 $m^2/g$, plus particulièrement 120 et 300 $m^2/g$.

13. Procédé selon la revendication 12, caractérisé en ce que ladite silice présente une surface CTAB comprise entre 120 et 400 $m^2/g$.

14. Procédé selon l'une quelconque des revendications 1 à 9, caractérisé en ce que ladite silice est du type bifonctionnelle et présente une surface BET comprise entre 80 et 200 $m^2/g$.

15. Procédé selon la revendication 14, caractérisé en ce que ladite silice présente une surface CTAB comprise entre 80 et 200 $m^2/g$.

16. Procédé selon l'une des revendications précédentes, caractérisé en ce que ladite silice présente une prise d'huile comprise entre 80 et 500 $cm^3/g$.

17. Procédé selon l'une des revendications précédentes, caractérisé en ce que ladite silice est une silice de précipitation.

18. Procédé selon l'une des revendications 1 à 17, caractérisé en ce qu'on réalise le traitement avec un zincate par mise en contact de la silice ou d'une suspension de silice avec un zincate.

**19.** Procédé selon l'une des revendications 1 à 18, caractérisé en ce que le traitement acide se fait par passage d'une solution acide sur la silice ou addition de cette solution à une suspension contenant la silice.

**20.** Procédé selon la revendication 19, caractérisé en ce que le traitement acide se fait par addition d'une solution acide à une suspension contenant la silice de manière que le pH de la suspension présente une valeur qui ne soit pas inférieure a 7 à la fin du traitement.

**21.** Procédé selon l'une des revendications 1 à 20 du type comprenant une réaction d'un silicate avec un agent acidifiant, l'obtention d'une suspension ou d'un gel de silice, une séparation et un séchage de la silice, caractérisé en ce qu'on traite avec un zincate le gâteau issu de la séparation de la silice de la suspension.

**22.** Procédé selon la revendication 21, caractérisé en ce qu'on traite le gâteau précité délité.

**23.** Procédé selon l'une des revendications 1 à 22, caractérisé en ce qu'on utilise un zincate alcalin.

**24.** Procédé selon l'une des revendications 1 à 23, caractérisé en ce qu'après le traitement par le zincate et le traitement acide on fait subir un mûrissement à la silice.

**25.** Composition dentifrice caractérisée en ce qu'elle comporte une silice préparée par le procédé selon l'une des revendications 1 à 24.

**26.** Composition dentifrice selon la revendication 25, caractérisée en ce qu'elle comporte du zinc.

**27.** Composition dentifrice selon la revendication 25 ou 26, caractérisée en ce qu'elle comporte au moins un élément choisi dans le groupe des fluorures, des phosphates, des polyphosphates, des polyphosphonates, des pyrophosphates, des guanidines.

**Claims**
**Claims for the following Contracting States : AT, BE, CH, DE, FR, GB, GR, IT, LI, LU, NL, SE**

**1.** Silica, compatible with zinc, characterised in that it comprises zinc at its surface and chemically bonded to this surface.

**2.** Silica according to Claim 1, characterised in that it gives rise only to a nil or negligible desorption of zinc when it is placed in an aqueous solution at pH 7.

**3.** Silica according to Claim 1 or 2, characterized in that it comprises Si-O-Zn groups at its surface.

**4.** Silica according to one of the preceding claims, characterized in that it has a compatibility with zinc of at least 50 %.

**5.** Silica according to Claim 4, characterized in that it has a compatibility with zinc of at least 80 %, more particularly of at least 95 %.

**6.** Silica according to one of the preceding claims, characterised in that it has a zinc content of not more than 10 %, more particularly not more than 5 %.

**7.** Silica according to Claim 6, characterised in that it has a zinc content of not more than 1 %, more particularly not more than 0.5 %.

**8.** Silica according to one of the preceding claims, characterized in that it has a BET surface of between 40 and 600 $m^2/g$.

**9.** Silica according to one of the preceding claims, characterized in that it has a CTAB surface of between 40 and 400 $m^2/g$.

**10.** Silica according to one of the preceding claims, of the abrasive type, characterised in that it has a BET surface of between 40 and 300 $m^2/g$.

**11.** Silica according to Claim 10, characterised in that it has a CTAB surface of between 40 and 100 $m^2/g$.

**12.** Silica according to any one of Claims 1 to 9, of the thickening type, characterised in that it has a BET surface of between 120 and 450 $m^2/g$, more particularly 120 and 300 $m^2/g$.

**13.** Silica according to Claim 12, characterised in that it has a CTAB surface of between 120 and 400 $m^2/g$.

**14.** Silica according to any one of Claims 1 to 9, of the difunctional type, characterised in that it has a BET surface of between 80 and 200 $m^2/g$.

**15.** Silica according to Claim 14, characterised in that it has a CTAB surface of between 80 and 200 $m^2/g$.

**16.** Silica according to one of the preceding claims, characterised in that it has an oil uptake of between 80 and 500 $cm^3/g$.

**17.** Silica according to one of the preceding claims, characterised in that it is a precipitated silica.

**18.** Process for obtaining a silica compatible with zinc, especially of the type according to one of the preceding claims, characterised in that silica, either prepared beforehand or during a stage of its preparation, is subjected to a treatment with a zincate and, jointly with or after this first treatment, to an acidic treatment.

**19.** Process according to Claim 18, characterised in that the treatment with a zincate is carried out by bringing silica or a suspension of silica into contact with a zincate.

**20.** Process according to either of Claims 18 and 19, characterised in that the acidic treatment is performed by passing an acidic solution over the silica or adding this solution to a suspension containing the silica.

**21.** Process according to Claim 20, characterised in that the acidic treatment is performed by adding an acidic solution to a suspension containing the silica so that the pH of the suspension has a value which is not lower than 7 at the end of the treatment.

**22.** Process according to one of Claims 18 to 21, of the type comprising a reaction of a silicate with an acidifying agent, obtaining a silica suspension or gel, separating and drying the silica, characterized in that the cake originating from the separation of the silica from the suspension is treated with a zincate.

**23.** Process according to Claim 22, characterised in that the slaked abovementioned cake is treated.

**24.** Process according to one of Claims 18 to 23, characterised in that an alkali metal zincate is employed.

**25.** Process according to one of Claims 18 to 24, characterized in that after the treatment with the zincate and the acidic treatment the silica is subjected to maturation.

**26.** Dentifrice composition characterized in that it comprises a silica of the type according to one of Claims 1 to 17 or of the type prepared by the process according to one of Claims 18 to 25.

**27.** Dentifrice composition according to Claim 26, characterized in that it comprises zinc.

**28.** Dentifrice composition according to Claim 26 or 27, characterized in that it comprises at least one component chosen from the group of fluorides, phosphates, polyphosphates, polyphosphonates, pyrophosphates and guanidines.

**Claims for the following Contracting State : ES**

1. Process for obtaining a silica compatible with zinc, comprising zinc at its surface and chemically bonded to this surface, characterised in that the silica, either prepared beforehand or during a stage of its preparation, is subjected to a treatment with a zincate and, jointly with or after this first treatment, to an acidic treatment.

2. Process according to Claim 1, characterised in that, when the said silica is placed in an aqueous solution at pH 7, it gives rise only to a nil or negligible desorption of zinc.

3. Process according to Claim 1 or 2, characterised in that the said silica comprises Si-O-Zn groups at its surface.

4. Process according to one of the preceding claims, characterised in that the said silica has a compatibility with zinc of at least 50 %.

5. Process according to Claim 4, characterised in that the said silica has a compatibility with zinc of at least 80 %, more particularly of at least 95 %.

6. Process according to one of the preceding claims, characterised in that the said silica has a zinc content of not more than 10 %, more particularly not more than 5 %.

7. Process according to Claim 6, characterised in that the said silica has a zinc content of not more than 1 %, more particularly of not more than 0.5 %.

8. Process according to one of the preceding claims, characterised in that the said silica has a BET surface of between 40 and 600 $m^2/g$.

9. Process according to one of the preceding claims, characterised in that the said silica has a CTAB surface of between 40 and 400 $m^2/g$.

10. Process according to one of the preceding claims, characterised in that the said silica is of the abrasive type and has a BET surface of between 40 and 300 $m^2/g$.

11. Process according to Claim 10, characterised in that the said silica has a CTAB surface of between 40 and 100 $m^2/g$.

12. Process according to any one of Claims 1 to 9, characterised in that the said silica is of the thickening type and has a BET surface of between 120 and 450 $m^2/g$, more particularly 120 and 300 $m^2/g$.

13. Process according to Claim 12, characterised in that the said silica has a CTAB surface of between 120 and 400 $m^2/g$.

14. Process according to any one of Claims 1 to 91 characterised in that the said silica is of the difunctional type and has a BET surface of between 80 and 200 $m^2/g$.

15. Process according to Claim 14, characterised in that the said silica has a CTAB surface of between 80 and 200 $m^2/g$.

16. Process according to one of the preceding claims, characterised in that the said silica has an oil uptake of between 80 and 500 $cm^3/g$.

17. Process according to one of the preceding claims, characterised in that the said silica is a precipitated silica.

18. Process according to one of Claims 1 to 17, characterised in that the treatment with a zincate is carried out by bringing the silica or a suspension of silica into contact with a zincate.

**19.** Process according to one of Claims 1 to 18, characterised in that the acidic treatment is performed by passing an acidic solution over the silica or adding this solution to a suspension containing the silica.

**20.** Process according to Claim 19, characterised in that the acidic treatment is performed by adding an acidic solution to a suspension containing the silica so that the pH of the suspension has a value which is not lower than 7 at the end of the treatment.

**21.** Process according to one of Claims 1 to 20, of the type comprising a reaction of a silicate with an acidifying agent, obtaining a silica suspension or gel, separating and drying the silica, characterised in that the cake originating from the separation of the silica from the suspension is treated with a zincate.

**22.** Process according to Claim 21, characterised in that the slaked abovementioned cake is treated.

**23.** Process according to one of Claims 1 to 22, characterised in that an alkali metal zincate is employed.

**24.** Process according to one of Claims 1 to 23, characterised in that, after the treatment with the zincate and the acidic treatment, the silica is subjected to maturing.

**25.** Dentifrice composition characterised in that it comprises a silica prepared by the process according to one of Claims 1 to 24.

**26.** Dentifrice composition according to Claim 25, characterised in that it comprises zinc.

**27.** Dentifrice composition according to Claim 25 or 26, characterised in that it comprises at least one component chosen from the group of fluorides, phosphates, polyphosphates, polyphosphonates, pyrophosphates and guanidines.

**Patentansprüche**
**Patentansprüche für folgende Vertragsstaaten : AT, BE, CH, DE, FR, GB, GR, IT, LI, LU, NL, SE**

**1.** Mit Zink verträgliche Kieselsäure, dadurch gekennzeichnet, daß sie an ihrer Oberfläche und an diese Oberfläche chemisch gebunden Zink enthält.

**2.** Kieselsäure nach Anspruch 1, dadurch gekennzeichnet, daß, wenn sie in eine wäßrige Lösung vom pH-Wert 7 gebracht wird, keine oder vernachläßigbare Zinkdesorption stattfindet.

**3.** Kieselsäure nach Anspruch 1 oder 2, dadurch gekennzeichnet, daß sie an ihrer Oberfläche Gruppierungen Si-O-Zn aufweist.

**4.** Kieselsäure nach einem der vorangehenden Ansprüche, dadurch gekennzeichnet, daß sie eine Verträglichkeit mit Zink von mindestens 50 % besitzt.

**5.** Kieselsäure nach Anspruch 4, dadurch gekennzeichnet, daß sie eine Verträglichkeit mit Zink von mindestens 80 % und insbesondere von mindestens 95 % besitzt.

**6.** Kieselsäure nach einem der vorangehenden Ansprüche, dadurch gekennzeichnet, daß sie einen Zinkgehalt von höchstens 10 %, insbesondere von höchstens 5 %, aufweist.

**7.** Kieselsäure nach Anspruch 6, dadurch gekennzeichnet, daß sie einen Zinkgehalt von höchstens 1 %, insbesondere von höchstens 0,5 %, aufweist.

**8.** Kieselsäure nach einem der vorangehenden Ansprüche, dadurch gekennzeichnet, daß sie eine BET-Oberfläche im Bereich von 40 bis 600 $m^2/g$ besitzt.

**9.** Kieselsäure nach einem der vorangehenden Ansprüche, dadurch gekennzeichnet, daß sie eine CTAB-Oberfläche im Bereich von 40 bis 400 $m^2/g$ besitzt.

10. Kieselsäure vom Schleifnitteltyp nach einer dar vorangehenden Ansprüche, dadurch gekennzeichnet, daß sie eine BET-Oberfläche im Bereich von 40 bis 300 $m^2/g$ aufweist.

11. Kieselsäure nach Anspruch 10, dadurch gekennzeichnet, daß sie eine CTAB-Oberfläche im Bereich von 40 bis 100 $m^2/g$ aufweist.

12. Kieselsäure vom Verdickungsmitteltyp nach einem der Ansprüche 1 bis 9,dadurch gekennzeichnet, daß sie eine BET-Oberfläche im Bereich von 120 bis 450 $m^2/g$, insbesondere von 120 bis 300 $m^2/g$, aufweist.

13. Kieselsäure nach Anspruch 12, dadurch gekennzeichnet, daß sie eine CTAB-Oberfläche im Bereich von 120 bis 400 $m^2/g$ aufweist.

14. Kieselsäure vom bifunktionellen Typ nach einem der Ansprüche 1 bis 9, dadurch gekennzeichnet, daß sie eine BET-Oberfläche im Bereich von 80 bis 200 $m^2/g$ aufweist.

15. Kieselsäure nach Anspruch 14, dadurch gekennzeichnet, daß sie eine CTAB-Oberfläche im Bereich von 80 bis 200 $m^2/g$ aufweist.

16. Kieselsäure nach einem der vorangehenden Ansprüche, dadurch gekennzeichnet, daß sie eine Ölaufnahme im Bereich von 80 bis 500 $cm^3/g$ aufweist.

17. Kieselsäure nach einem der vorangehenden Ansprüche, dadurch gekennzeichnet, daß es sich um eine Fällungskieselsäure handelt.

18. Verfahren zur Herstellung einer mit Zink verträglichen Kieselsäure, vor allem des Typs gemäß einem der vorangehenden Ansprüche, dadurch gekennzeichnet, daß man die Kieselsäure - entweder zuvor hergestellt oder im Verlauf einer Stufe ihrer Herstellung - einer Behandlung mit einem Zinkat und gleichzeitig oder nach dieser ersten Behandlung einer Säurebehandlung unterwirft.

19. Verfahren nach Anspruch 18, dadurch gekennzeichnet, daß man die Behandlung mit einem Zinkat ausführt, indem man die Kieselsäure oder eine Kieselsäuresuspension mit einem Zinkat in Berührung bringt.

20. Verfahren nach einem der Ansprüche 18 oder 19, dadurch gekennzeichnet, daß die Säurebehandlung erfolgt, indem eine Säurelösung über die Kieselsäure geleitet oder die Säurelösung zu einer Suspension, die Kieselsäure enthält, zugegeben wird.

21. Verfahren nach Anspruch 20, dadurch gekennzeichnet, daß die Säurebehandlung durch Zugabe einer Säurelösung zu einer Suspension, die Kieselsäure enthält, in der Weise erfolgt, daß die Suspension am Ende der Behandlung einen pH-Wert von nicht weniger als 7 aufweist.

22. Verfahren nach einem der Ansprüche 18 bis 21 des Typs, der eine Reaktion eines Silicats mit einem ansäuernden Mittel, den Erhalt einer Suspension oder eines Kieselsäuregels, ein Abtrennen und ein Trocknen der Kieselsäure umfaßt, dadurch gekennzeichnet, daß man den bei der Abtrennung der Kieselsäure von der Suspension erhaltenen Kuchen mit einem Zinkat behandelt.

23. Verfahren nach Anspruch 22, dadurch gekennzeichnet, daß man den genannten Kuchen in fragmentiertem Zustand behandelt.

24. Verfahren nach einem der Ansprüche 18 bis 23, dadurch gekennzeichnet, daß man ein Alkalizinkat verwendet.

25. Verfahren nach einem der Ansprüche 18 bis 24, dadurch gekennzeichnet, daß man die Kieselsäure nach der Behandlung mit dem Zinkat und nach der Behandlung mit der Säure reifen läßt.

26. Zahnpflegemasse, dadurch gekennzeichnet, daß sie eine Kieselsäure vom Typ gemäß einem der Ansprüche 1 bis 17 oder vom Typ, hergestellt durch das Verfahren nach einem der Ansprüche 18 bis

25, enthält.

**27.** Zahnpflegemasse nach Anspruch 26, dadurch gekennzeichnet, daß sie Zink enthält.

**28.** Zahnpflegemasse nach Anspruch 26 oder 27, dadurch gekennzeichnet, daß sie mindestens ein Element, ausgewählt aus der Gruppe der Fluoride, Phosphate, Polyphosphate, Polyphosphonate, Pyrophosphate und Guanidine, enthält.

**Patentansprüche für folgenden Vertragsstaat : ES**

**1.** Verfahren zur Herstellung einer mit Zink verträglichen Kieselsäure, die an ihrer Oberfläche und an diese Oberfläche chemisch gebunden Zink enthält, dadurch gekennzeichnet, daß man die Kieselsäure - entweder zuvor hergestellt oder im Verlauf einer Stufe ihrer Herstellung - einer Behandlung mit einem Zinkat und gleichzeitig oder nach dieser ersten Behandlung einer Säurebehandlung unterwirft.

**2.** Verfahren nach Anspruch 1, dadurch gekennzeichnet, daß, wenn die Kieselsäure in eine wäßrige Lösung vom pH-Wert 7 gebracht wird, keine oder vernachläßigbare Zinkdesorption stattfindet.

**3.** Verfahren nach Anspruch 1 oder 2, dadurch gekennzeichnet, daß die Kieselsäure an ihrer Oberfläche Gruppierungen Si-O-Zn aufweist.

**4.** Verfahren nach einem der vorangehenden Ansprüche, dadurch gekennzeichnet, daß die Kieselsäure eine Verträglichkeit mit Zink von mindestens 50 % besitzt.

**5.** Verfahren nach Anspruch 4, dadurch gekennzeichnet, daß die Kieselsäure eine Verträglichkeit mit Zink von mindestens 80 % und insbesondere von mindestens 95 % besitzt.

**6.** Verfahren nach einem der vorangehenden Ansprüche, dadurch gekennzeichnet, daß die Kieselsäure einen Zinkgehalt von höchstens 10 %, insbesondere von höchstens 5 %, aufweist.

**7.** Verfahren nach Anspruch 6, dadurch gekennzeichnet, daß die Kieselsäure einen Zinkgehalt von höchstens 1 %, insbesondere von höchstens 0,5 %, aufweist.

**8.** Verfahren nach einem der vorangehenden Ansprüche, dadurch gekennzeichnet, daß die Kieselsäure eine BET-Oberfläche im Bereich von 40 bis 600 $m^2/g$ besitzt.

**9.** Verfahren nach einem der vorangehenden Ansprüche, dadurch gekennzeichnet, daß die Kieselsäure eine CTAB-Oberfläche im Bereich von 40 bis 400 $m^2/g$ besitzt.

**10.** Verfahren nach einem der vorangehenden Ansprüche, dadurch gekennzeichnet, daß die Kieselsäure dem Schleifnitteltyp angehört und eine BET-Oberfläche im Bereich von 40 bis 300 $m^2/g$ aufweist.

**11.** Verfahren nach Anspruch 10, dadurch gekennzeichnet, daß die Kieselsäure eine CTAB-Oberfläche im Bereich von 40 bis 100 $m^2/g$ aufweist.

**12.** Verfahren nach einem der Ansprüche 1 bis 9, dadurch gekennzeichnet, daß die Kieselsäure dem Verdickungsmitteltyp angehört und eine BET-Oberfläche im Bereich von 120 bis 450 $m^2/g$, insbesondere von 120 bis 300 $m^2/g$, aufweist.

**13.** Verfahren nach Anspruch 12, dadurch gekennzeichnet, daß die Kieselsäure eine CTAB-Oberfläche im Bereich von 120 bis 400 $m^2/g$ aufweist.

**14.** Verfahren nach einem der Ansprüche 1 bis 9, dadurch gekennzeichnet, daß die Kieselsäure dem bifunktionellen Typ angehört und eine BET-Oberfläche im Bereich von 80 bis 200 $m^2/g$ aufweist.

**15.** Verfahren nach Anspruch 14, dadurch gekennzeichnet, daß die Kieselsäure eine CTAB-Oberfläche im Bereich von 80 bis 200 $m^2/g$ aufweist.

16. Verfahren nach einem der vorangehenden Ansprüche, dadurch gekennzeichnet, daß die Kieselsäure eine Ölaufnahme im Bereich von 80 bis 500 $cm^3/g$ aufweist.

17. Verfahren nach einem der vorangehenden Ansprüche, dadurch gekennzeichnet, daß es sich um eine Fällungskieselsäure handelt.

18. Verfahren nach einem der Ansprüche 1 bis 17, dadurch gekennzeichnet, daß man die Behandlung mit einem Zinkat ausführt, indem man die Kieselsäure oder eine Kieselsäuresuspension mit einem Zinkat in Berührung bringt.

19. Verfahren nach einem der Ansprüche 1 bis 18, dadurch gekennzeichnet, daß die Säurebehandlung erfolgt, indem eine Säurelösung über die Kieselsäure geleitet oder die Säurelösung zu einer Suspension, die Kieselsäure enthält, zugegeben wird.

20. Verfahren nach Anspruch 19, dadurch gekennzeichnet, daß die Säurebehandlung durch Zugabe einer Säurelösung zu einer Suspension, die Kieselsäure enthält, in der Weise erfolgt, daß die Suspension am Ende der Behandlung einen pH-Wert von nicht weniger als 7 aufweist.

21. Verfahren nach einem der Ansprüche 1 bis 20 des Typs, der eine Reaktion eines Silicats mit einem ansäuernden Mittel, den Erhalt einer Suspension oder eines Kieselsäuregels, ein Abtrennen und ein Trocknen der Kieselsäure umfaßt, dadurch gekennzeichnet, daß man den bei der Abtrennung der Kieselsäure von der Suspension erhaltenen Kuchen mit einem Zinkat behandelt.

22. Verfahren nach Anspruch 21, dadurch gekennzeichnet, daß man den genannten Kuchen in fragmentiertem Zustand behandelt.

23. Verfahren nach einem der Ansprüche 1 bis 22, dadurch gekennzeichnet, daß man ein Alkalizinkat verwendet.

24. Verfahren nach einem der Ansprüche 1 bis 23, dadurch gekennzeichnet, daß man die Kieselsäure nach der Behandlung mit dem Zinkat und nach der Behandlung mit der Säure reifen läßt.

25. Zahnpflegemasse, dadurch gekennzeichnet, daß sie eine Kieselsäure, hergestellt gemäß einem der Ansprüche 1 bis 24, enthält.

26. Zahnpflegemasse nach Anspruch 25, dadurch gekennzeichnet, daß sie Zink enthält.

27. Zahnpflegemasse nach Anspruch 25 oder 26, dadurch gekennzeichnet, daß sie mindestens ein Element, ausgewählt aus der Gruppe der Fluoride, Phosphate, Polyphosphate, Polyphosphonate, Pyrophosphate und Guanidine, enthält.